# EUROPEAN PATENT APPLICATION

(11) **EP 0 835 633 A2**
(43) Date of publication of application: **15.04.1998**
(21) Application number: 97307209.3
(22) Date of filing: 17.09.1997
(51) Int. Cl.: A61B 5/0255, A61B 5/025

(54) **Apparatus and system for pulse diagnosis and the method thereof**

(30) Priority: 18.09.1996 JP 246624/96
(71) Applicant: Maruya, Tokinori, Tokyo (JP)
(72) Inventor: Maruya, Tokinori, Tokyo (JP)
(74) Representative: Downey, William Gerrard

(57) **Abstract**

It is an object of the present invention to provide an apparatus which automatically detects pulse waves from a patient, and allows a high-precision pulse diagnosis on the basis of those wave data.

This object is achieved by an apparatus whose operation includes: winding pulse sensor belts around both wrists of a patient; detecting pressure signals associated with pulses from left and right radial arteries through pulse sensors 2b-2d; converting those signals into pulse waves through a wave transformer 8; adjusting the height of pulse waves through a wave level adjuster 11; comparing the adjusted pulse waves with a plurality of symptomatic waves stored in a symptomatic wave storing memory 9 through a comparator, and choosing the symptomatic wave corresponding with or similar to the pulse wave; and extracting the disease condition data corresponding to the symptomatic wave from a disease condition data storing memory 10 through a disease condition data extractor 13. A heart beat counter 15 provides heart beat counts on the basis of pulse waves. The apparatus stores the chosen symptomatic wave, pulse wave, disease condition data and heart beat count into a patients' data storing memory 14, and provides, as output, the same data to a monitor 4 or a printer 5 for display or print-out.

## Description

### BACKGROUND OF THE INVENTION

### Field of the Invention

The present invention relates generally to an apparatus and system for pulse diagnosis and a method thereof, and more particularly to a technique which can be profitably applied to automatic execution of pulse diagnosis based on Chinese medicine.

### Related Art Statement

According to the investigation by the present inventor, in Chinese medicine, the physician, in addition to verbal examination and observation of the patient's tongue, feels his/her pulsation which reflects his/her cardiovascular state, diagnoses the disease and prescribes a medicine appropriate for the treatment of the disease.

Normally, for feeling the patient's pulse, the physician uses his both hands, one for each of the patient's wrists: he applies the index, middle and ring fingers of one hand to the three sites of patient's wrist where pulsation of radial artery is most manifest.

It was found, however, by the present inventor that this method of pulse feeling presents with following problems.

That is, the method tends to give results wide in variation and low in precision, which are sometimes misleading. This is because this method is practiced by the physician himself, and thus heavily depends on the personal skill of the physician. Therefore, the result therefrom vary widely depending on how much a given physician is accustomed to the technique.

### SUMMARY OF THE INVENTION

It is an object of the present invention is to provide an apparatus and system for pulse diagnosis which automatically detects the pulse wave of a patient, and which gives a highly precise diagnosis based on the pulse wave analysis, and a method of pulse diagnosis based on such apparatus and system.

The typical outline of the invention disclosed in the present application will be briefly described as follows.

That is, the pulse diagnosis apparatus of the present invention comprises: a pulse detecting means for recording pulses from the radial artery at a specified site as electrical signals; a pulse diagnosis means for converting the electrical signals recorded by the pulse detecting means into pulse waves, for choosing symptomatic waves corresponding with or similar to those pulse waves from the stored data, and for extracting disease condition data corresponding to the chosen symptomatic waves; and an output means for providing the pulse waves recorded by the pulse detecting means, the corresponding symptomatic waves and the disease condition data as an output.

The present invention further includes a pulse diagnosis apparatus wherein the pulse diagnosis means comprises: a wave transformer for converting the electrical signals recorded by the pulse detecting means into pulse waves; a first memory for storing symptomatic waves necessary for diagnosis; a second memory for storing various disease condition data corresponding to the symptomatic waves; a wave level adjuster for adjusting pulse waves produced by the wave transformer so that they may have the same sizes as the symptomatic waves stored in the first memory; a comparator for comparing the adjusted pulse waves with the symptomatic waves stored in the first memory and for choosing the symptomatic waves corresonding with or similar to the pulse waves recorded; and a disease condition data extractor for extracting from the second memory the disease condition data which are related with the symptomatic waves chosen by the comparator as most closely correlated with the pulse waves.

The pulse diagnosis apparatus of the present invention further includes, within the pulse diagnosis means, a third memory for storing at least one of the following three kinds of data: the pulse waves whose sizes have been adjusted by the wave level adjuster, pulse wave data treated by the comparator, and the disease condition data extracted by the disease condition data extractor.

The pulse diagnosis apparatus of the present invention still further includes, within the pulse diagnosis means, a counter which counts heart beats based on the pulse waves produced by the wave transformer.

The pulse diagnosis system of the present invention comprises: a plurality of pulse diagnosis terminals each of which includes a pulse detecting means for recording pulses from the radial artery at a specified site as electrical signals, and an output means for providing as an output pulse waves, symptomatic waves and disease condition data which are inputted; and a pulse diagnosis means connected with the plurality of pulse diagnosis terminals on a real time basis, for converting the electrical signals provided by the pulse detecting means into the pulse waves, for choosing the symptomatic waves corresponding with or similar to the pulse waves, and for extracting the disease condition data corresponding with the chosen symptomatic waves.

The present invention further includes a pulse diagnosis system wherein the pulse diagnosis means comprises: a wave transformer for converting the electrical signals recorded by the pulse detecting means into pulse waves; a first memory for storing symptomatic waves necessary for diagnosis; a wave level adjuster for adjusting the pulse waves produced by the wave transformer so that they may have the same sizes as the symptomatic waves stored in the first memory; a second memory for storing various disease condition data corresponding to the symptomatic waves; a comparator for comparing the adjusted pulse waves with the symptomatic waves stored in the first memory and for choosing the symptomatic waves corresponding with or similar to the pulse waves recorded; a disease condition data extractor for extracting from the second memory the disease condition data which are related with the symptomatic waves chosen by the comparator as most closely correlated with the pulse waves recorded.

The pulse diagnosis system of the present invention further includes, within the pulse diagnosis means, a third memory for storing at least one of the following three kinds of data: the pulse waves whose sizes have been adjusted by the wave level adjuster, pulse wave data treated by the comparator, and the disease condition data extracted by the disease condition data extractor.

The pulse diagnosis system of the present invention still further includes, within the pulse diagnosis means, a counter which counts heart beats based on the pulse waves produced by the wave transformer.

The method for pulse diagnosis of the present invention comprises: a step of detecting pulses from the radial artery at a specified site through a pulse detecting means and of converting the detected pulses into electrical signals; a step of converting the electrical signals recorded by the pulse detecting means into pulse waves through a wave transformer; a step of adjusting the pulse waves produced by the wave transformer through a wave level adjuster so that they may have the same sizes as symptomatic waves necessary for diagnosis which are stored ina first memory; a step of comparing the pulse waves adjusted by the wave level adjuster with the symptomatic waves stored in the first memory through a comparator and of choosing the symptomatic waves corresponding with or similar to the pulse waves; a step of extracting through a disease condition data extractor the disease condition data corresponding with the symptomatic waves chosen by the comparator from a second memory to store the disease condition data; and a step of providing as an output the pulse waves recorded, the corresponding symptomatic waves and the disease condition data to an output means.

The method for pulse diagnosis of the present invention further includes a step of storing into a third memory at least one of the following three kinds of data: the pulse waves whose sizes have been adjusted by the wave level adjuster, pulse wave data treated by the comparator, and the disease condition data extracted by the disease condition data extractor.

The method for pulse diagnosis of the present invention still further includes a step of counting heart beats based on the pulse waves produced by the wave transformer.

The storage medium of the present invention stores: a step of detecting pulses from the radial artery at a specified position and of transforming the detected pulses into electrical signals; a step of converting the electrical signals into pulse waves; a step of counting heart beats based on the pulse waves; a step of adjusting the pulse waves so that they may have the same sizes as symptomatic waves necessary for diagnosis; a step of comparing the adjusted pulse waves with the symptomatic waves stored in advance; and a step of choosing the symptomatic waves corresponding with or similar to the pulse waves.

The storage medium of the present invention further stores a step of extracting the disease condition data corresponding with the chosen symptomatic waves.

The storage medium of the present invention still further stores a step of providing as an output the pulse waves recorded, the symptomatic waves and the heart beat counts.

As is evident from above, the apparatus, system and method of the present invention allow an automated recording of pulses from a patient and indication of the pulse waves of the patient, the correlated symptomatic waves and the disease condition data. Consequently, the apparatus, system and method of the present invention give a stable, high-precision pulse diagnosis.

Other features and advantages of the present invention will become readily apparent from the following written description of the present specification and the accompanying drawings.

### BRIEF DESCRIPTION OF THE DRAWINGS

Fig. 1 illustrates how a preferred embodiment of the pulse diagnosis apparatus of the present invention is constituted.

Fig. 2 illustrates where the pulse sensors of a preferred embodiment of the present invention are applied for the detection of pulses.

Fig. 3 is a block diagram of the pulse diagnosis apparatus of a preferred embodiment of the present invention.

Fig. 4 is a flowchart illustrating how data are analyzed in the pulse diagnosis apparatus of a preferred embodiment of the present invention.

Fig. 5(a) is an example of a list of relationships between symptomatic pulses and underlying diseases whose data are stored in the memory of the system of a preferred embodiment of the present invention.

Fig. 5(b) is another example of a list of relationships between symptomatic pulses and underlying diseases whose data are stored in the memory of the system of a preferred embodiment of the present invention.

Fig. 6 gives some examples of disease condition data stored in the memory of the system of a preferred embodiment of the present invention.

Fig. 7 illustrates how the pulse diagnosis system of another preferred embodiment of the present invention is constituted.

### DESCRIPTION OF THE PREFERRED EMBODIMENT

Preferred embodiments of the present invention will now be described in detail with reference to the drawings.

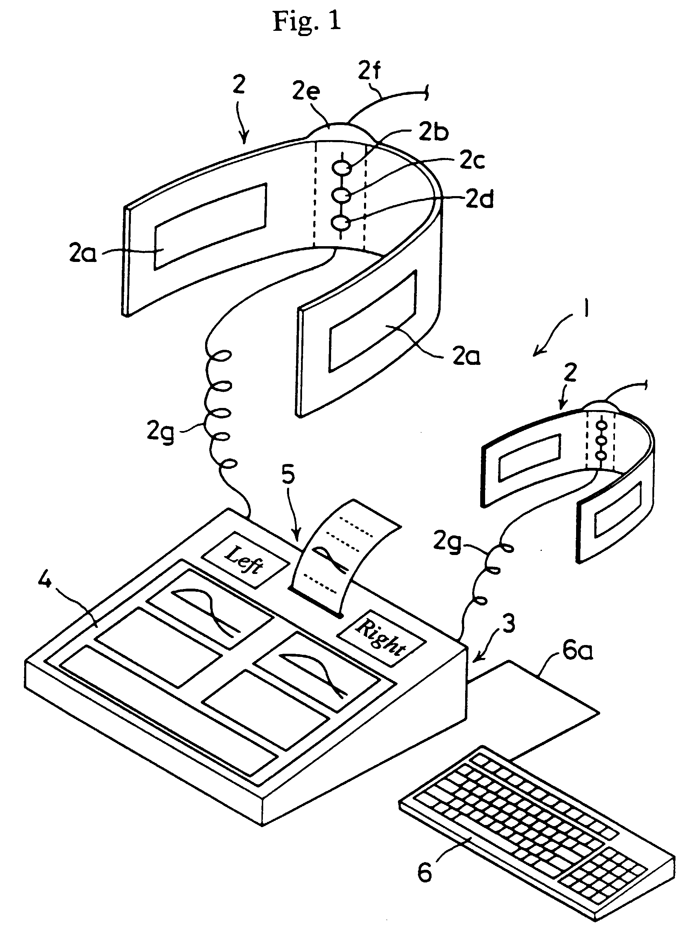

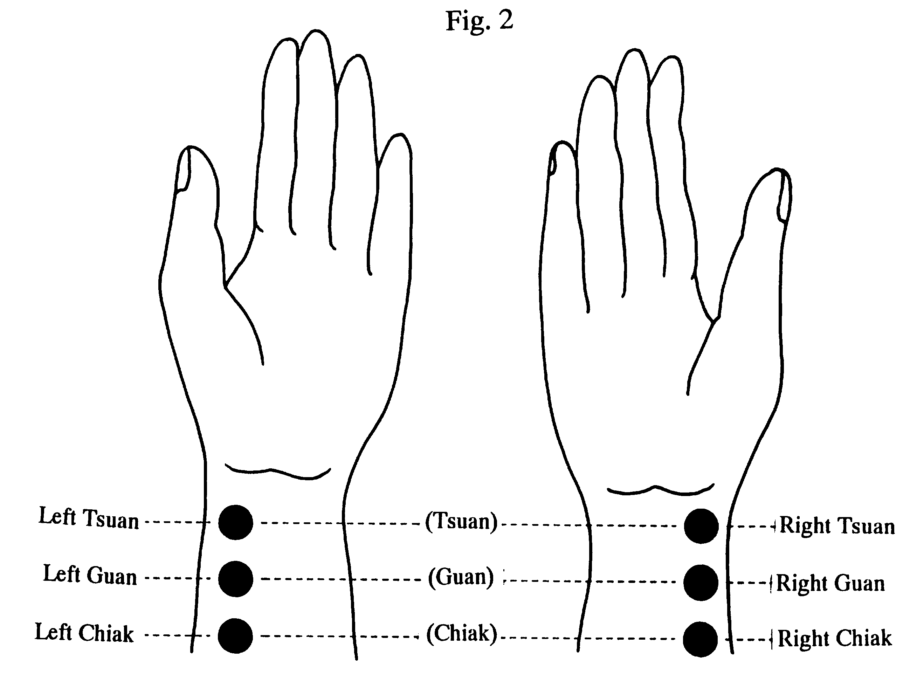

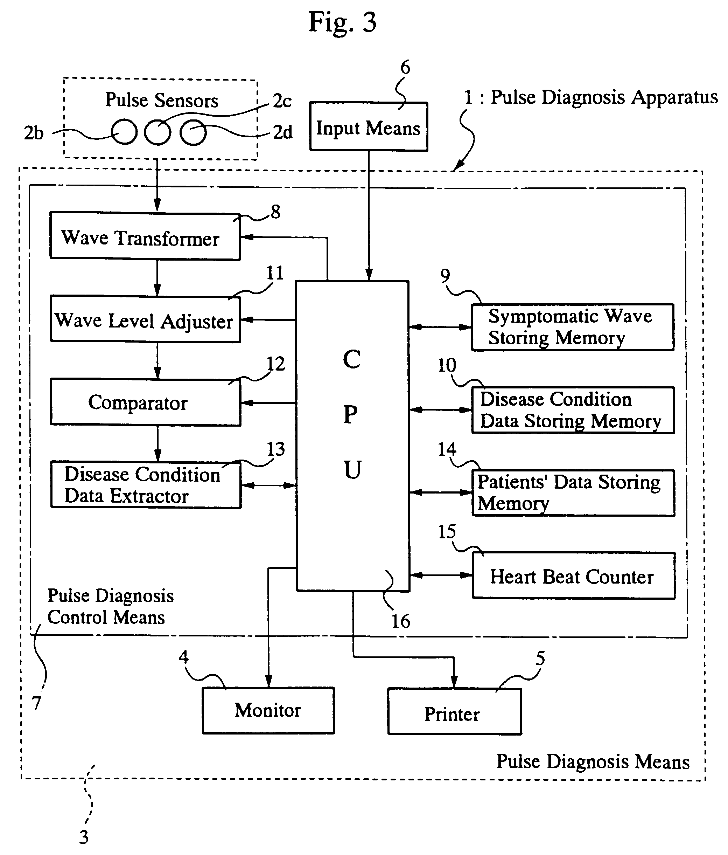

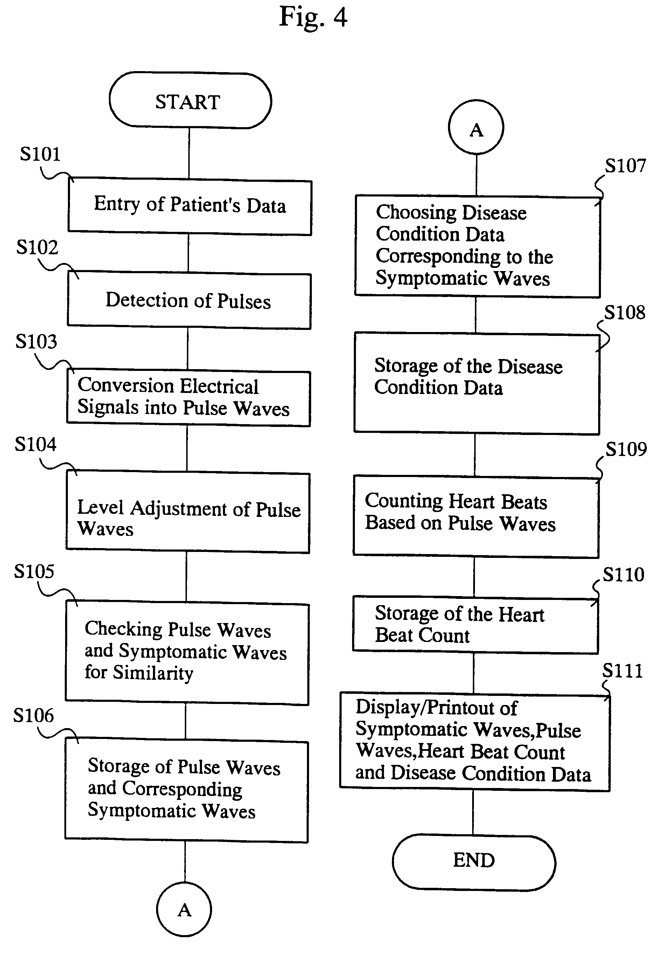

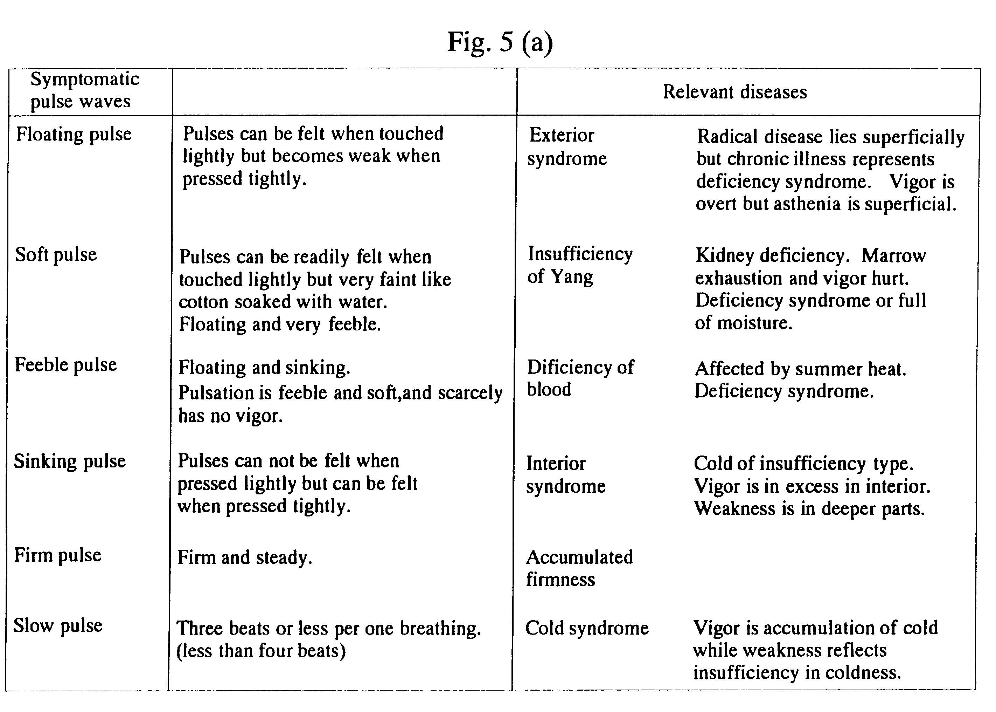

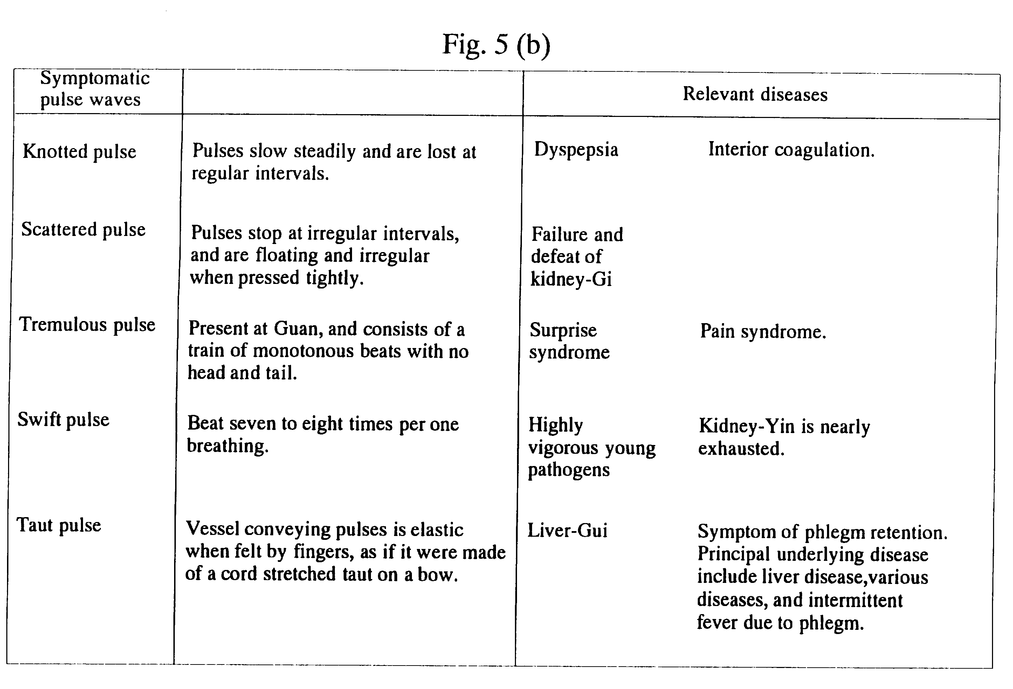

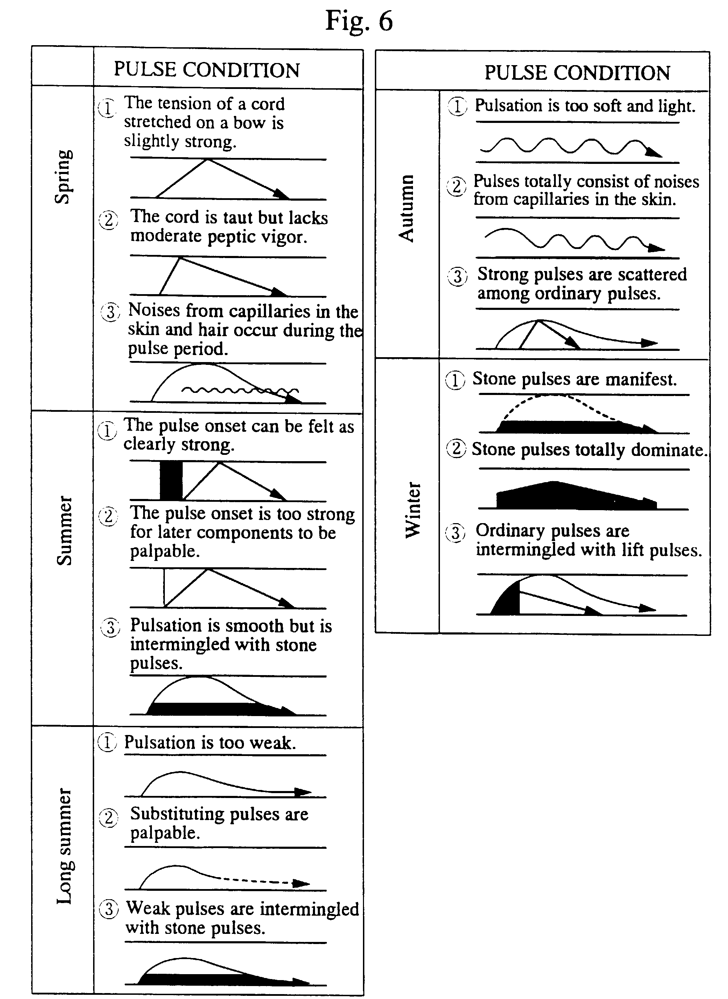

Fig. 1 illustrates how a preferred embodiment of the pulse diagnosis apparatus of the present invention is constituted; Fig. 2 illustrates where the pulse sensors of a preferred embodiment of the present invention are applied for the detection of pulses; Fig. 3 is a block diagram of the pulse diagnosis apparatus of a preferred embodiment of the present invention; Fig. 4 is a flowchart illustrating how data are analyzed in the pulse diagnosis apparatus of a preferred embodiment of the present invention; Figs. 5(a) and 5(b) are examples of a list of relationships between symptomatic pulses and underlying diseases whose data are stored in the memory of the system of a preferred embodiment of the present invention; and Fig. 6 gives some examples of disease condition data stored in the memory of the system of a preferred embodiment of the present invention.

In this embodiment, a pulse diagnosis apparatus 1 which assists pulse diagnosis by detecting pulses from the radial artery is provided with band-like pulse sensor belts 2 which are wound around specified positions (ports to Tsuan) of both wrists of the patient which will give most conspicuous pulsation of the radial artery.

The pulse diagnosis apparatus 1 is provided with surface fasteners 2a which are mounted close to the ends of each pulse sensor belt 2, and fasten the pulse sensor belt 2 to fix it to a site of one wrist of the patient beneath which runs the radial artery.

At the center of the pulse sensor belt 2 are placed three pulse sensors (pulse detecting means) 2b-2d comprising an array of three pressure sensors.

These three pulse sensors 2b-2d are placed on three different points along the course of superficial radial artery which gives the most conspicuous pulsation: Tsuan (the most distal point of superficial radial artery), Guan (the middle point of the same artery) and Chiak (the most proximal point of the same artery) . Guan corresponds to the most elevated part close to palm (styloid processus of the radius), and Tsuan is situated distal to Guan while Chiak is proximal to Guan.

Generally, human has the above-described three points or Tsuan, Guan and Chiak on each hand, and these points are called left Tsuan, left Guan and left Chiak when they are in the left hand, while the corresponding points in the right hand are called right Tsuan, right Guan and right Chiak.

As shown in Fig. 1, the pulse sensor belt 2 is further provided with an air bag 2e close to the site where the array of pulse sensors 2b-2d are located. The air bag 2e, when inflated by the air sent through an elastic tube 2f made of rubber from a pump connected with the tube 2f, gives a pressure to the pulse sensors 2b-2d to allow them to come into firm contact with left Tsuan, Guan and Chiak and right Tsuan, Guan and Chiak.

The pulse diagnosis apparatus 1 is further provided with a pulse diagnosis means 3 which is electrically connected with the pulse sensors 2b-2d of the pulse sensor belt 2 through a cable 2g. The pulse diagnosis means 3 includes a pulse diagnosis control means described later which concerns with all controls and calculations executed in the pulse diagnosis apparatus 1, a monitor 4 (output means) which displays diagnosis results on the screen, and a printer 5 (output means) which prints diagnosis results on paper.

The pulse diagnosis apparatus 1 is still further provided with an input means 6 such as a keyboard through which one can feed any data at will. This input means 6 is electrically connected through another cable 6a to the pulse diagnosis means 3.

The pulse diagnosis control means 7 installed in the pulse diagnosis means 3 includes, as shown in Fig. 3, a wave transformer 8 which converts pulses recorded by the pulse sensors 2b-2d into pulse waves, and transmits electrical signals provided by the pulse sensors 2b-2d to the wave transformer 8.

The pulse diagnosis control means 7 is further provided with a symptomatic wave storing memory (first memory) 9 which consists, for example, of a ROM (Read Only Memory) and stores symptomatic waves necessary for pulse diagnosis, or normalized wave data corresponding with various pulse conditions.

The pulse diagnosis control means 7 is provided with a disease condition data storing memory (second memory) 10 which consists, similarly to the first one, of a ROM and stores disease condition data including names, courses and underlying causes.

The pulse diagnosis control means 7 is further provided with a wave level adjuster 11 which adjusts the pulse waves provided by the wave transformer 8 so that those waves may have the same height as the symptomatic waves stored in the symptomatic wave storing memory 9, and is so connected as to receive the pulse waves provided by the wave transformer 8.

The pulse diagnosis control means 7 is further provided with a comparator 12 which compares the pulse waves whose height has been adjusted by the wave level adjuster, with symptomatic waves stored in the symptomatic wave storing memory 9, and chooses the symptomatic waves corresponding with or similar to the pulse waves, and is so connected as to receive the pulse waves whose height has been adjusted by the wave level adjuster 11.

The pulse diagnosis control means 7 is still further provided with a disease condition data extractor 13 which extracts, from the disease condition data storing memory 10, disease condition data corresponding with the symptomatic waves chosen by the comparator 12.

The pulse diagnosis control means 7 stores the pulse waves whose height has been adjusted by the wave level adjuster 11, symptomatic wave data chosen by the comparator, and disease condition data extracted by the disease condition data extractor 13. These data are stored, for example, in a patients' data storing memory (third memory) 14 such as RAM (Random Access Memory) .

The pulse diagnosis control means 7 is still further provided with a heart beat counter 15 which counts heart beats based on the pulse waves provided by the wave transformer 8.

The pulse diagnosis control means 7 is still further provided with a CPU which is connected to all those elements including the wave transformer 8, symptomatic wave storing memory 9, disease condition data storing memory 10, wave level adjuster 11, comparator 12, disease condition data extractor 13, patients' data storing memory 14 and heart beat counter 15, and controls them all.

Feeding data into or fetching data from the symptomatic wave storing memory 9, disease condition data storing memory 10 and patients' data storing memory 14, delivering data to a monitor 4 in the pulse diagnosis means 3 for display, feeding data to a printer 5 for print-out, and feeding input through an input means 6 to the system--all these processes take place through CPU 16.

The operation of those embodiments of the present invention will be given below with reference to Fig. 4 which presents a flowchart of processes necessary for the operation of pulse diagnosis apparatus 1 represented in Figs. 1-3.

Before applying a pulse diagnosis on a patient, the operator must feed necessary data through the input means 6 to a computer (step S101).

The necessary data include the name and ID number of the patient. The patient's data is delivered through CPU 16 to the patients' data storing memory 14 for storage. The stored data can be easily retrieved on account of the ID number, and utilized for the reference of previous diagnosis results or for the revision of those data on reexamination of the same patient.

Then, the operator winds the pulse sensor belts 2 around the wrists of patient such that the pulse sensors 2b-2d are securely placed opposite to the left Tsuan, Guan and Chiak, and to the right Tsuan, Guan and Chiak, and fastens the belts there with the surface fastener 2a.

The operator pushes a start button to activate the pump which delivers air into the air bag 2e until a specified pressure is reached in that bag. Then, pulse diagnosis starts and proceeds for a specified interval, say, for about five minutes.

Pressure signals associated with pulses and detected by the pulse sensors 2b-2d (step S102) are delivered to the wave transformer 8 of the pulse diagnosis control means 7 in the pulse diagnosis means 3.

The pressure signals associated with pulses are converted by the wave transformer into pulse waves (step S103), which are then delivered to the wave level adjuster 11. The pulse waves are so adjusted by the wave level adjuster 11 as to give a height comparable to that of various symptomatic waves stored in the symptomatic wave storing memory 9 (step S104). The thus adjusted pulse waves are delivered to the comparator 12.

At this step, if pressure signals recorded by the pulse sensors 2b-2d are so large in magnitude that they exceed the adjustment capacity of wave level adjuster 11, recourse may be made to the following measure. The pump is put into action manually or by a certain automatic mechanism and adjusted to lower the pressure of air sent to the air bag 2e. On the contrary, if pressure signals are too low in height to be properly treated by the wave level adjuster 11, the pump may be put into action manually or by the same automatic mechanism to elevate the pressure of air sent to the air bag 2e to a proper level.

The comparator 12 compares the pulse waves whose height has been adjusted, with various symptomatic waves retrieved by CPU 16 from the symptomatic wave storing memory 9, choose the symptomatic waves identical to or most closely similar to the pulse waves (step S105), and stores the thus chosen symptomatic waves by way of CPU 16 into the patients' data storing memory 14, together with the pulse waves whose height has been adjusted by the wave level adjuster 11 (step S106)

Later, the disease condition data extractor 13 searches, by way of CPU 16, among various disease condition data stored in the disease condition data storing memory 10, the data that correspond to the symptomatic waves chosen by the comparator 12 (step S107), finds them, and stores them into the patients' data storing memory 14 (step S108).

A few examples of various symptomatic wave data stored in the symptomatic wave storing memory 9 and of disease condition data stored in the disease condition data storing memory 10 are presented in Figs. 5 and 6, respectively, for illustration. Fig. 6 gives a list of symptomatic waves together with their names, characteristics and relations with various diseases. In addition, other data including disease names and their underlying causes are similarly stored in the same memory.

Assignment of data to the memories may be made as follows: the patients' data storing memory 14 stores only pulse wave and symptomatic wave data, and CPU 16 gets access to the disease condition data storing memory 10, only when it must display or print the disease condition data corresponding to the pulse wave recorded.

The heart beat counter 15 chooses relevant pulse waves stored in the patients' data storing memory 14, and delivers them to CPU 16 which counts heart beats on the basis of those data (step S109), and stores the result in the patients' data storing memory (step S110).

Pulse diagnosis requires, as noted earlier, about five minutes, and, during this period, processes of step S102 to S108 are repeated.

On completion of pulse diagnosis, the pulse waves, symptomatic waves, disease condition data and heart beat count retrieved from the patients' data storing memory are displayed on the monitor 4, and printed on paper with the printer 5 (step S111).

Display of pulse waves and symptomatic waves may take place at the same time, or in succession one after the other.

In this embodiment of the present invention, the pulse diagnosis apparatus 1 automatically detects pulses of a patient, analyze them, correlate them with most probable symptomatic waves and disease condition data, and displays or prints those pulse waves, symptomatic waves and relevant disease condition data on the monitor 4 or on paper with the printer 5. Thus, the diagnosis is independent of the skill of the operator engaged in pulse diagnosis, and stable, high-precision diagnosis is ensured.

The embodiment described above concerns with the pulse diagnosis apparatuses 1 installed in individual pharmacies or drug stores which deal with diagnosis data independently from each other. Those pharmacies and drug stores (dealers) may be connected under a special contract to a clinics center (center) to form a network system. Thus, a plurality of peripheral machines at dealers are put under the control of a host computer at the center to form a pulse diagnosis system.

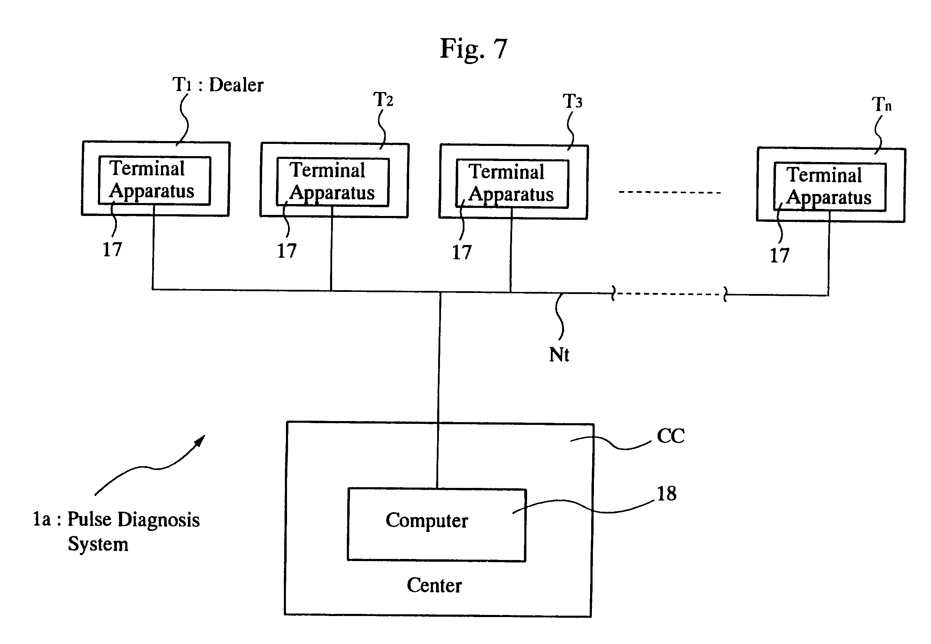

Such pulse diagnosis system la includes, as shown Fig. 7, terminal apparatus (pulse diagnosis terminal) 17 installed at dealers T1-Tn, each of which consists of pulse sensor belts 2, monitor 4, printer 5 and input means 6, and a host computer (pulse diagnosis means) 18 placed at a clinics center or the headquarters of network which consists of a pulse diagnosis control means 7 as is shown in Fig. 1.

The terminal apparatus 17 are connected through public or private communication lines Nt with the computer 18 on a real time basis.

This network system allows individual dealers T1-Tn to dispense with the use of computers 18, and thus to greatly reduce the cost and space necessary for the installment of those diagnosis machines.

The invention achieved by the present inventor has been specifically described based on preferred embodiments according to the invention. The present invention, however, should not necessarily be limited to the above-described embodiments, and, needless to say, can be modified variously within the scope not departing from the technical gist.

For example, the embodiment includes pulse sensor belts each incorporating three sensors corresponding to three sensitive spots Tsuan, Guan and Chiak of a patient so as to achieve a high-precision recording of the pulse waves. The pulse sensor belt may be modified so as to contain one or two sensors instead of three to apply them to one or two of the three sensitive spots. The apparatus with such sensor unit will be able to achieve practically the same effective pulse diagnosis with the apparatus of the present invention.

The principal advantages obtained by the invention disclosed in the present application can be summarized as follows.
(1) The pulse diagnosis apparatus of the present invention picks up pulses of a patient, analyzes them, compares them with symptomatic waves, and presents correlative disease condition data on display automatically.
(2) A plurality of pulse diagnosis terminals are connected with a central pulse diagnosis means to form a network system on a real time basis. This system allows a great reduction in cost and space required for installment of pulse diagnosis machines.
(3) The two advantages describe above ensures a stable, high-precision diagnosis, independent of the skill of the operator.

## Claims

1. A pulse diagnosis apparatus which comprises:
a pulse detecting means for recording pulses from a radial artery at a specified site as electrical signals;
a pulse diagnosis means for converting the electrical signals recorded by the pulse detecting means into pulse waves, for choosing symptomatic waves corresponding with or similar to the pulse waves, and for extracting disease condition data corresponding to the chosen symptomatic waves; and
an output means for providing the pulse waves recorded, the corresponding symptomatic waves and the disease condition data outputted by the pulse diagnosis means as an output.

2. A pulse diagnosis apparatus as set forth in claim 1 wherein the pulse diagnosis means comprises:
a wave transformer for converting the electrical signals recorded by the pulse detecting means into pulse waves;
a first memory for storing symptomatic waves necessary for diagnosis;
a second memory for storing various disease condition data corresponding to the symptomatic waves;
a wave level adjuster for adjusting the pulse waves produced by the wave transformer so that they may have the same sizes as the symptomatic waves stored in the first memory;
a comparator for comparing the adjusted pulse waves with the symptomatic waves stored in the first memory and for choosing the symptomatic waves corresponding with or similar to the pulse waves recorded; and
a disease condition data extractor for extracting from the second memory the disease condition data corresponding to the symptomatic waves chosen by the comparator.

3. A pulse diagnosis apparatus as set forth in claim 2 which further includes, within the pulse diagnosis means, a third memory for storing at least one of the data consisting of the pulse waves whose sizes have been adjusted by the wave level adjuster, pulse wave data treated by the comparator, and the disease condition data extracted by the disease condition data extractor.

4. A pulse diagnosis apparatus as set forth in claim 2 which further includes, within the pulse diagnosis means, a counter which counts heart beats based on the pulse waves produced by the wave transformer.

5. A pulse diagnosis apparatus as set forth in claim 3 which further includes, within the pulse diagnosis means, a counter which counts heart beats based on the pulse waves produced by the wave transformer.

6. A pulse diagnosis system which comprises:
a plurality of pulse diagnosis terminals each of which includes a pulse detecting means for recording pulses from a radial artery at a specified site as electrical signals, and an output means for providing as an output pulse waves, symptomatic waves and disease condition data which are inputted; and
a pulse diagnosis means connected with the plurality of pulse diagnosis terminals on a real time basis, for converting the electrical signals provided by the pulse detecting means into the pulse waves, for choosing the symptomatic waves corresponding with or similar to the pulse waves, for extracting the disease condition data corresponding with the chosen symptomatic waves, and for providing those data as an output.

7. A pulse diagnosis system as set forth in claim 6 wherein the pulse diagnosis means comprises:
a wave transformer for converting the electrical signals recorded by the pulse detecting means into pulse waves;
a first memory for storing symptomatic waves necessary for diagnosis;
a wave level adjuster for adjusting the pulse waves produced by the wave transformer so that they may have the same sizes as the symptomatic waves stored in the first memory;
a second memory for storing various disease condition data corresponding to the symptomatic waves;
a comparator for comparing the adjusted pulse waves with the symptomatic waves stored in the first memory and for choosing the symptomatic waves corresponding with or similar to the pulse waves recorded; and
a disease condition data extractor for extracting from the second memory the disease condition data correspongin to the symptomatic waves chosen by the comparator.

8. A pulse diagnosis system as set forth in claim 7 which further includes, within the pulse diagnosis means, a third memory for storing at least one of the data consisting of the pulse waves whose sizes have been adjusted by the wave level adjuster, pulse wave data treated by the comparator, and the disease condition data extracted by the disease condition data extractor.

9. A pulse diagnosis system as set forth in claim 7 which still further includes, within the pulse diagnosis means, a counter which counts heart beats based on the pulse waves produced by the wave transformer.

10. A pulse diagnosis system as set forth in claim 8 which still further includes, within the pulse diagnosis means, a counter which counts heart beats based on the pulse waves produced by the wave transformer.

11. Amethod for pulse diagnosis which comprises:
a step of detecting pulses from a radial artery at a specified site by a pulse detecting means and of converting the detected pulses into electrical signals;
a step of converting the electrical signals recorded by the pulse detecting means into pulse waves by a wave transformer;
a step of adjusting the pulse waves produced by the wave transformer by a wave level adjuster so that they may have the same sizes as symptomatic waves stored in a first memory necessary for diagnosis;
a step of comparing the pulse waves adjusted by the wave level adjuster with the symptomatic waves stored in the first memory by a comparator and of choosing the symptomatic waves corresponding with or similar to the pulse waves recorded;
a step of extracting by a disease condition data extractor the disease condition data corresponding with the symptomatic waves chosen by the comparator from a second memory to store the disease condition data; and
a step of providing as an output the pulse waves recorded, the corresponding symptomatic waves and the disease condition data by an output means.

12. A method for pulse diagnosis as set forth in claim 11 which further includes a step of storing into a third memory at least one of the data consisting of the pulse waves whose sizes have been adjusted by the wave level adjuster, pulse wave data treated by the comparator, and the disease condition data extracted by the disease condition data extractor.

13. Amethod for pulse diagnosis as set forth in claim 11 which further includes a step of counting heart beats based on the pulse waves produced by the wave transformer.

14. Amethod for pulse diagnosis as set forth in claim 12 which further includes a step of counting heart beats based on the pulse waves produced by the wave transformer.

15. A storage medium for storing a computer program, said computer program comprising:
a step of detecting pulses from a radial artery at a specified site and of converting the detected pulses into electrical signals;
a step of converting the electrical signals into pulse waves;
a step of counting heart beats based on the pulse waves;
a step of adjusting the pulse waves so that they may have the same sizes as symptomatic waves necessary for diagnosis;
a step of comparing the adjusted pulse waves with the symptomatic waves stored in advance; and
a step of choosing the symptomatic waves corresponding with or similar to the pulse waves.

16. A storage medium as set forth in claim 15, wherein said computer program further comprising a step of extracting the disease condition data corresponding with the chosen symptomatic waves.

17. A storage medium as set forth in claim 16, wherein said computer program further comprising a step of providing as an output the pulse waves recorded, the corresponding symptomatic waves and the heart beat counts.
